# EUROPEAN PATENT SPECIFICATION

(11) **EP 2 440 123 B1**
(45) Date of publication and mention of the grant of the patent: **19.02.2020**
(21) Application number: 10719715.4
(22) Date of filing: 17.05.2010
(51) Int. Cl.: A61B 5/022

(54) **BLOOD PRESSURE CUFF**
BLUTDRUCKMANSCHETTE
BRASSARD DE TENSIOMÈTRE

(30) Priority: 19.05.2009 US 468438
(43) Date of publication of application: 18.04.2012
(73) Proprietor: WELCH ALLYN, INC., Skaneateles Falls, NY 13153 (US)
(72) Inventor: VIVENZIO, Robert, L., Auburn, NY 13021 (US); EDWARDS, Ian, K., Skaneateles, NY 13152 (US); LIA, Raymond, A., Auburn, NY 13021 (US); PERKINS, Jeff, Tully, NY 13159 (US); KARLA, Sean, Syracuse, NY 13215 (US)
(74) Representative: Findlay, Alice Rosemary
(86) International application number: PCT/US2010/035062
(87) International publication number: WO 2010/135216

(56) References cited:
- WO-A1-2007/116588
- WO-A1-2007/125546
- DE-A1- 2 220 233
- US-A- 1 729 297
- US-A- 6 149 600
- US-A1- 2001 005 777
- US-A1- 2004 181 156
- US-A1- 2006 293 600

## Description

### FIELD OF THE APPLICATION

This application generally relates to the field of medical diagnostic instruments and more specifically to a low cost, ecologically friendly blood pressure sleeve or cuff that is biodegradable or capable of being recycled.

### BACKGROUND OF THE INVENTION

Sphygmomanometers are commonly known and established medical diagnostic instruments used for measuring patient blood pressure. In one well known version, a reusable cuff or sleeve made from fluid-impermeable material is wrapped about the limb (e.g., arm or leg) of the patient. Various sized sleeves are made, depending on the class (i.e., child, adult, neonatal) of the patient. Most sleeves of this type are defined by either a pair of planar sheets that are sealed together or are formed from a single sheet, the sleeve either having a contained bladder or an inflatable interior compartment. These sleeves further typically include hook and loop fasteners disposed at specific locations on opposing sides in order to permit releasable and adjustable attachment to and removal of the sleeve from a patient. The bladder or interior inflatable compartment is inflated using pneumatic means, such as a pump, which is tethered to the cuff by means of a flexible hose attached to a barb that is provided on the exterior of the sleeve. Pressure variations in the sleeve can then be detected by a gage housing having a dial indicator that is attached to the cuff. In mechanical versions, the gage housing contains a movement mechanism having a pressure responsive element, such as a diaphragm, wherein pressure variations are imparted to a dial indicator on the gage housing, according to the well- known oscillometric technique. Electronic blood pressure measuring versions, which may or may not include a pump directly within the gage housing, are also known, such as those manufactured by Welch Allyn, Inc. and Omron Corporation, among others, the results being displayed for example, using an LCD. In the latter types of devices, either the oscillometric (pulsatile) method or the auscultatory method of pressure measurement can be utilized, the latter being used in combination with a stethoscope or microphone.

These diagnostic instruments are repletely found in a doctor's office or within examination rooms within a medical facility or a hospital. With regard to a medical facility or hospital and depending upon the number of procedures that are performed on a patient during an examination or a typical hospital or urgent care visit, there are reasons why a blood pressure sleeve should not be reused, for example, the potential for cross contamination of infectious fluids between patients, among others.
For example, the documents US 6 149 600 A and WO 2007/116558 A1 each disclose a blood pressure cuff .

Therefore, there is a need presently to inexpensively provide a disposable blood pressure sleeve, without degrading quality or accuracy in measurement or the use of same.

In the course of developing a sleeve that is disposable, additional consideration must be made with regard to environmental/ ecological issues, including landfill, emission and other related concerns.

### SUMMARY OF THE INVENTION

The invention is defined in the appended independent claims 1 and 8.

In one version, the sleeve is manufactured from at least one sheet of a suitable lightweight material that is also fluid-impermeable, such as polypropylene, the at least one polypropylene sheet having a coated side and an uncoated nonwoven side, the at least one sheet being formed into the sleeve, including the inflatable portion.

According to the invention the sleeve is configured to be releasably attachable onto the limb of a patient in overlaying relation, the sleeve having a slotted portion opposite an extending port such that the protruding port extends through the slotted portion when the sleeve is wrapped about the limb. The slotted portion provides a limit to the range of limb sizes to which the cuff can be attached. For example, various child/adult and neonatal cuff versions can be provided. The sleeve can be secured to the patient for wrapping such as by adhesives, clips, hook and loop fasteners or using other releasable means. In one embodiment, the port and the slotted portions are centered along the width dimension of the cuff, enabling the cuff to be attached more generally about the limb of a patient as opposed to presently used cuffs.

The extending port according to one version is a socket that is configured to receive at least one of a hose adapter and a gage housing that is used to inflate the sleeve through pneumatic means, such as a bulb or pump wherein the sleeve is readily adaptable for use in connection with either mechanical and/or electronic blood pressure measurement apparatus. The socket is configured to permit the at least one of a hose adapter and a gage housing engaged in the socket to be rotatable about an axis of the socket.

In one version, means are provided that can prevent the sleeve from being reused. In one such version, hook and loop fasteners are provided as closure means for the cuff in which the sleeve material itself serves as the function of loop fasteners and in which the attachability (i.e., the shear strength) of the hook fasteners to the loop fasteners degrades with each use, thereby rendering the sleeve incapable of intended use after one or a finite number of uses. Alternatively, other disabling means can be provided depending on the closure means, such as a tear strip that destroys the sleeve and preventing the sleeve from being rewrapped following removal from the patient.

According to another version, there is provided a disposable blood pressure cuff, said cuff comprising a sleeve having an interior; and an inflatable portion within at least a portion of said sleeve interior, the cuff including a port that directly and fluidly interconnects with the interior of said inflatable portion, said cuff being biodegradable. In one version, the cuff is made from a material that is treated with an additive that renders said cuff biodegradable after a predetermined time period. According to one version, the cuff is made from at least one of polypropylene and polyethylene.

According to the invention there is provided a blood pressure cuff comprising a flexible sleeve including an inflatable portion, a port extending from an exterior surface of the inflatable portion and a slotted portion sized for receiving the port when the sleeve is circumferentially wrapped about the limb of a patient.

The slotted portion is sized in order to limit the range of limb sizes to which the cuff can be attached. In one version, the slotted portion and port are aligned with one another, each being disposed at substantially the center of the width dimension of the sleeve. Advantageously, the cuff can be wrapped in at least two opposite orientations about the limb of a patient and in which the port is in the same location relative to the patient in each orientation.

According to the invention, there is provided a method for manufacturing a blood pressure cuff, comprising the steps defined in claim 8.

In one version, the sleeve member is partially or entirely made from a recyclable material, such as polypropylene. In another version, the sleeve member is made from a biodegradable material such as polypropylene or polyethylene.

In one version, the sleeve member is treated with an additive enabling the sleeve member to be biodegradable after a predetermined time period.

In another version, the sleeve member is provided with closure means for releasably securing the sleeve in a wrapped configuration. The closure means in one embodiment can include hook and loop fasteners in which the exterior surface of the sleeve is made from a material that provides adhesion to a hook fastener portion of the sleeve, thereby serving as loop fasteners.

According to another aspect not forming part of the invention, there is provided a method for sequentially dispensing blood pressure cuffs, said method comprising the steps of providing a plurality of blood pressure cuffs in a stacked relation. Each of the cuffs are made from a highly flexible fluid impermeable material in which dispensing of same by pulling a portion of a cuff and pulling the first cuff from the stack as contained within an enclosure that allows the cuffs to be removed one at a time and in which each succeeding cuff is advanced for sequential dispense within the container.

In one exemplary version, a box-like enclosure having a dispense slot disposed therein is sized to receive a portion of each stacked cuff or sleeve, enabling each said sleeve to be pulled sequentially therefrom and in which the sleeves are either biodegradable or recyclable.

In another version, the cuffs can be packaged in other forms for ready dispense, such as in roll form.

One advantage provided by the herein described apparatus is that an ecologically friendly blood pressure cuff can be manufactured and dispensed for use at relatively low cost. In at least one version and to enable closure of the cuff about the patient, hook and loop fasteners can be employed. However, by having the sleeve made from a material that enables adhesion to a typical hook fastener, there is no need to specifically incorporate loop fasteners in the sleeve. Moreover, by providing an inherent loop fastener in combination with the slotted portion, enables the attachment point to be further from the edge of the sleeve, thereby not only improving adhesion, but also minimizing the required adhesion area between the respective hook and loop fasteners. Moreover, the material of the sleeve can be selected such that adhesion of the sleeve material forming the inherent loop fasteners degrades with each attachment and detachment from the patient, the sleeve will no longer be useful within a finite number of applications.

Another advantage using a blood pressure cuff as described herein is that cross-contamination between patients can be effectively minimized.

Yet another advantage is that the herein described sleeves, though disposable in nature, do not significantly degrade the environment or cause ecological issues. One factor for making an ecologically friendly, yet disposable cuff is through the homogeneity of material used in the manufacture of the sleeves. By manufacturing the cuff, including the port, entirely from a single material readily and advantageously permits recyclability of this product. Even if the port is not made from the precise material, the cuff still is highly capable of being adequately recycled. Selection of a suitable material such as polypropylene or polyethylene further permits the cuff to be incinerated without any toxic byproduct emissions, as found for example, in the case of known polyvinylchloride (PVC) cuffs. Alternatively, the cuff can be made from a combination of materials, such as polyethylene and polypropylene, that are treated with at least one additive that enables the cuff to be biodegradable after a predetermined time period.

An advantage of the herein described invention is that the slotted portion creates a range finder for limiting the size limb of patient to which the sleeve can be attached. The slotted portion in combination with the external port further provides additional securement of the sleeve while the sleeve portion also acts as a guide to insure the sleeve is properly wrapped about the limb of a patient.

Yet another advantage realized by the present sleeve is that the port and slotted portion can be positioned "on center" with respect to the sleeve; that is, each can be positioned at substantially the center of the width dimension of the sleeve. This positioning permits the sleeve to be attached without having to first position the sphygmomanometer port relative to the patient, other than with respect to the brachial artery. That is to say, the direction of wrap of the cuff becomes inconsequential and therefore simplifies use. Moreover and due to this symmetrical location of the port, the port can be utilized as the arterial marker.

Still another advantage is that the herein described sleeves or cuffs can be easily stored and packaged, as well as easily and readily dispensed thereby handling inventory/storage issues.

The herein described sleeve through its choice of material(s) is lighter weight and less stiff than previously known cuffs, including previously known disposable or single patient blood pressure cuffs, and is also more comfortable for the patient as it is worn.

These and other features and advantages will become readily apparent from the following Detailed Description, which should be read in conjunction with the accompanying drawings.

### BRIEF DESCRIPTION OF THE DRAWINGS

FIG. 1 is a partially exploded view of a disposable blood pressure cuff according to a first embodiment of the present disclosure;
FIG. 2 is a partially assembled view of the disposable blood pressure cuff of Fig. 1 ;
FIG. 3 is a top perspective view of the disposable blood pressure cuff of Figs. 1 and 2;
FIG. 4 is a top perspective view of the disposable blood pressure cuff of Figs. 1-3 shown in a wrapped condition, the cuff having a port connector attached to the port of the cuff;
FIG. 5 is a top plan view of a blood pressure cuff made in accordance with a second embodiment;
FIG. 6 is a top perspective view of the disposable blood pressure cuff of Fig. 5;
FIG. 7 illustrates the disposable blood pressure cuff of Figs. 5 and 6, as wrapped about a limb of a patient;
FIG. 8 is a perspective view of a disposable blood pressure cuff made in accordance with another embodiment;
FIG. 9 is a perspective view of the disposable blood pressure cuff of Fig. 8, shown in a partially wrapped condition;
FIGS. 10 and 11 illustrate views of the disposable blood pressure cuff of Figs. 8 and 9 as wrapped about the limb of a patient, demonstrating that the cuff can be wrapped for use in more than one orientation; and
FIG. 12 is a top perspective view of a container packaging a plurality of stacked disposable blood pressure cuffs.

### DETAILED DESCRIPTION

The following description relates to several exemplary embodiments of a disposable, single use or single patient blood pressure cuff or sleeve, as well as packaging for dispensing the sleeves and related methods for dispensing and packaging the disposable sleeves. It will be readily apparent, however, that a number of other variations and modifications embodying the concepts described herein are possible. In addition, certain terms such as, "top", "bottom", "upper", "lower", "above", "below", "over", "beneath" and the like are used throughout in order to provide a suitable frame of reference with regard to the accompanying drawings. These terms, however, are not intended to be overlimiting, except where so specifically noted.

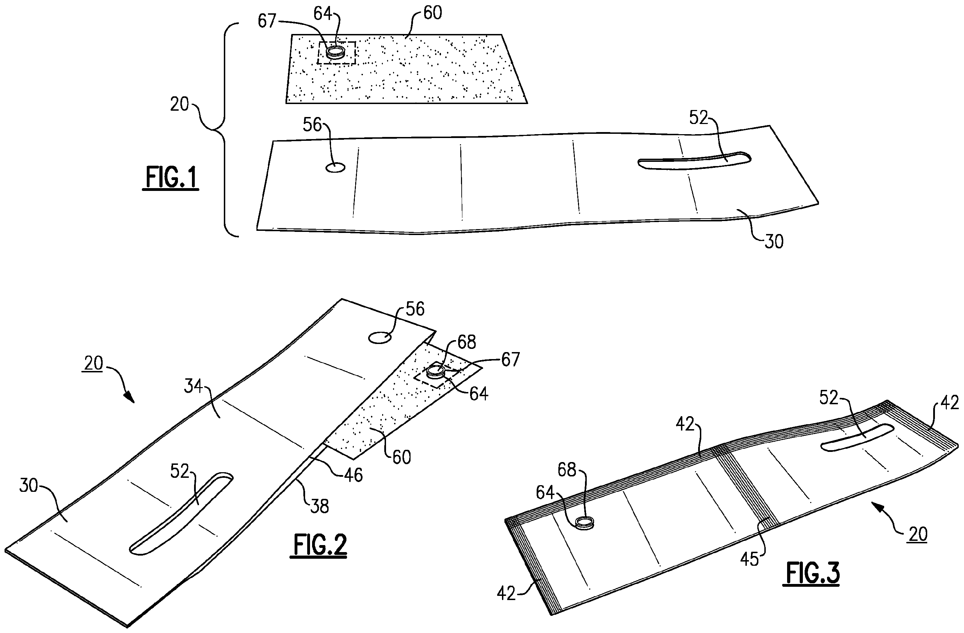

Referring to Figs. 1-3, there is shown a disposable blood pressure cuff 20 that is made in accordance with a first embodiment. The cuff 20 is defined by a highly flexible sleeve member 30, which according to this exemplary embodiment is made from a thin cellulosic material such as paper. The sleeve member 30 is formed by suitable means and according to this specific version, is constructed from a mailing envelope, though other suitable configurations can easily be imagined. Preferably, a stabilizing mesh made from fiber is preferably incorporated within the flexible sleeve member 30 to aid in wearability. Other suitable materials can be utilized, provided these materials are preferably tear-resistant, including certain so-called "green" or environmentally friendly, including recyclable materials such as polypropylene as described in greater detail in a later embodiment. For example and alternatively, the sleeve member can also be made from a material such as polyethylene and treated with an additive that causes the sleeve member to become biodegradable within a predetermined time interval (e.g., 2-3 months). Exemplary additives that are useful for this purpose include Green Solutions PDI BD-0701 or Oxo-Degrader.

The envelope-like structure of the flexible sleeve member 30, according to this exemplary version, is made up of a single sheet of material that is folded along one edge to define a pair of planar sleeve portions 34, 38, each sleeve portion having a length dimension that is significantly larger than a corresponding width dimension. The envelope-like structure is created by sealing the remaining edges 42, Fig. 3, of the sleeve 30 by heat sealing, ultrasonic or RF welding or other suitable means. Alternatively, the cuff 20 can be made from multiple sheets and sealed along all peripheral edges thereof. A slotted region 52 is formed through each of the sleeve portions 34, 38 on one side of the flexible sleeve 30, while a circular opening 56 is provided through an opposite side thereof through one of the planar sheets 34.

According to this exemplary version, an inflatable bladder 60, shown in Figs. 1, 2, is placed within the interior 46 of the side of the sleeve 30 having the circular opening 56 prior to sealing of the peripheral edge 42, Fig. 3. Alternatively, an inflatable portion of the flexible sleeve member 30 can be sealed on all edges, including an interior bordering edge 45, Fig. 3, thereof as described in a later embodiment. An exterior port is provided herein in the form of a socket 64 that extends from the bladder 60 and communicates fluidly with the interior thereof. The socket 64 is defined herein as an open-ended cylindrical cavity having a circumferential lip 67, which according to this embodiment extends from the exterior of the bladder 60 and is sized to extend through the circular opening 56 of the sleeve member 30. It will be readily apparent that the opening 56 can assume other shapes depending on the socket or port that is used therewith; for example, a hexagonal or other suitably shaped opening and port could be utilized. An opening 68 within the socket 64 extending into the interior of the bladder 60. As a result, the socket 64 enables fluid as well as mechanical interconnection with blood pressure measuring apparatus including a gage housing or hose adapter by way of a releasable snap-fitting connection. Additional details concerning a suitable socket design are provided in US 6 422 086 B1.

The bladder 60 according to this embodiment is defined by a fluid-impermeable and flexible material and is further defined by a substantially rectangular configuration. It should be noted, however, that this shape should not be limiting, meaning other suitable geomteries can be easily utilized. The bladder 60 can be inflated, as described in greater detail below by pneumatic means, such as a pump or bulb (not shown) attached preferably in releasable fashion to the socket 64.

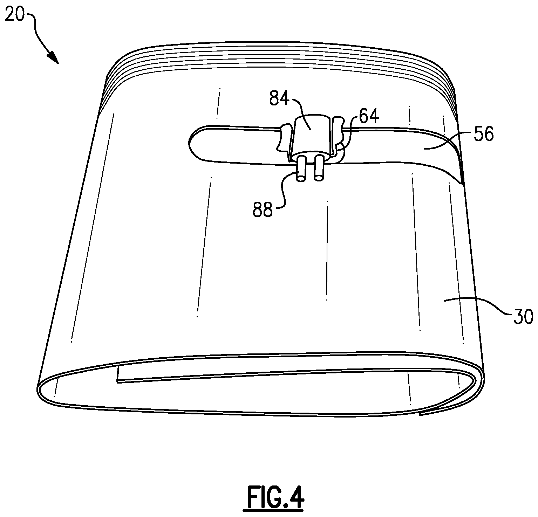

The slotted portion 52, as noted above, extends through each of the sleeve portions 34, 38 and along the major dimension of the sleeve member 30 opposite that of the bladder 60. The major dimension of the slotted portion 52 is aligned with the port 56 and is configured, as shown in Fig. 4, to provide a means of limiting the degree of wrapping of the cuff 20 about the limb of a patient (not shown) when the sheets are looped. As a result, the range of arm sizes that the cuff can be used is limited. The sleeve 30 can be retained in a wrapped form, such as by means of an adhesive, hook and loop fasteners, clips or other suitable attachment means.

As shown in Fig. 4, the cuff 20 can receive pneumatic means and be wrapped about the limb of a patient (not shown). The sleeve member 30 can be equipped with hooks, clips or other fastening means such as hook and loop fasteners (not shown) provided on opposite sides of the sleeve member 30. In this instance, a connector or adapter 84 is releasably snap-fitted to the socket 64, this connector enabling the sleeve 30 to be attached to a plurality of blood pressure measuring devices by means of lumen connectors 88, this connector being more thoroughly described inUS 2006/293600 A1. According to another version, the engagement end of a gage housing (not shown) can be snap-fitted directly to the socket 64, Fig. 1, thereby mechanically and fluidly connecting a movement mechanism contained within the gage housing with the sleeve member 30. The gage housing can support either of a mechanical or electronic blood pressure gauge.

Following use by a patient/caregiver, the cuff 20 can be removed from the patient and discarded. Alternatively, the cuff 20 can be used in connection with a single patient, for example, over the course of a patient visit or a typical hospital stay. As described below, means can be provided for disabling the cuff 20 from being wrapped either once the cuff is removed or after a predetermined number of patient uses.

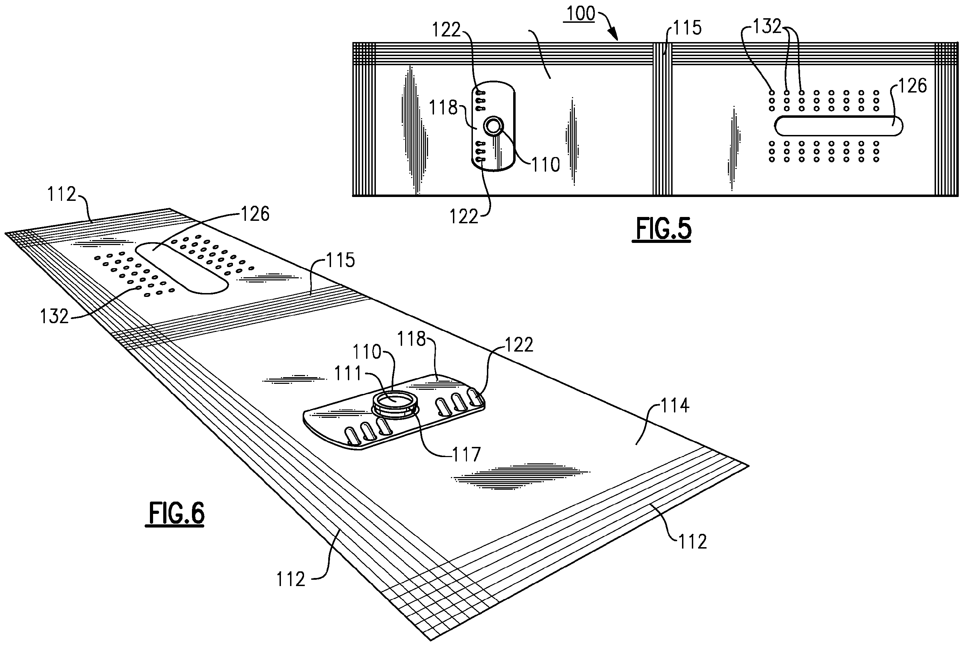

Referring to Figs. 5 and 6, there is shown a disposable blood pressure cuff 100 made in accordance with a second exemplary embodiment. The cuff 100 according to this version is defined by a flexible sleeve member 102 that is made from a durable, flexible and fluid- impermeable material such as polyethylene or polypropylene, which in the case of the former can be treated with an additive that enables the sleeve member to become biodegradable after a predetermined and finite period of time. Exemplary additives that permits biodegradability include PPI Green Solutions Additive, Oxo Degrader, among others. Alternatively and in the case of polypropylene, the entire cuff can be made therefrom, enabling recyclability as described in greater detail in a succeeding embodiment. According to this embodiment, the cuff 100 is formed from a single planar sheet formed into a folded configuration, the peripheral edges 112 of which are welded, bonded or otherwise sealed together in order to define a sleeve-like structure. Additionally, an intermediate transverse edge 112 is also sealed, thereby creating a sealed inflatable compartment 114 in lieu of a bladder. The material used according to this embodiment is fluid-impermeable and sufficiently flexible and resilient to permit expansion/inflation.

A small circular opening is cut into one of the planar sheets 104 of the formed cuff 100 over which is disposed a port or socket 110, the latter of which includes a circumferential or annular lip 117. According to this embodiment, the socket 110 is made from a flexible material such as polypropylene or polyethylene. The socket 110 according to this embodiment extends above the exterior surface of the planar sheet 104 and includes an opening 111 which extends into the interior of the inflatable portion 114 enabling fluid interconnection. The socket 110 is attached to a support structure 118 that is bonded or is otherwise sealed, according to this embodiment, to the exterior of the planar sheet 104. Alternatively, the support structure can be welded to the interior of the planar sheet with the socket extending directly through the opening. A plurality of angled protrusions 122 extend in spaced linear relation on opposing sides of the socket 110 on the exterior of the support structure 118. The protrusions 122 are also preferably made from a plastic material, such as polypropylene or polyethylene. The inflatable portion 114 of the sleeve member 102 is sealed on all four sides or edges 112, 115 by means of heat sealing, RF or ultrasonic welding or other appropriate techniques that provide a fluid-impermeable seal, wherein the material of at least the inflatable portion of the sleeve is made from a fluid-impermeable material and is flexible to enable expansion for inflation thereof by pneumatic means (not shown).

On the opposite side of the sleeve member 102 from the inflatable portion 114, a slotted portion 126 is provided having a major dimension aligned with the major dimension (length) of the sleeve member. The slotted portion 126 extends through each of the planar sheets 104, 108 defining the sleeve member 102 and is used in conjunction with the socket 114 to control the range of limb sizes to which the cuff 100 can be utilized. A series of small openings 132 are disposed in spaced linear fashion along each lateral side of the slotted portion 126 and substantially along the length thereof, the openings each being sized to retain a raised protrusion 122 and the slotted portion 126 being sized to retain the socket 110.

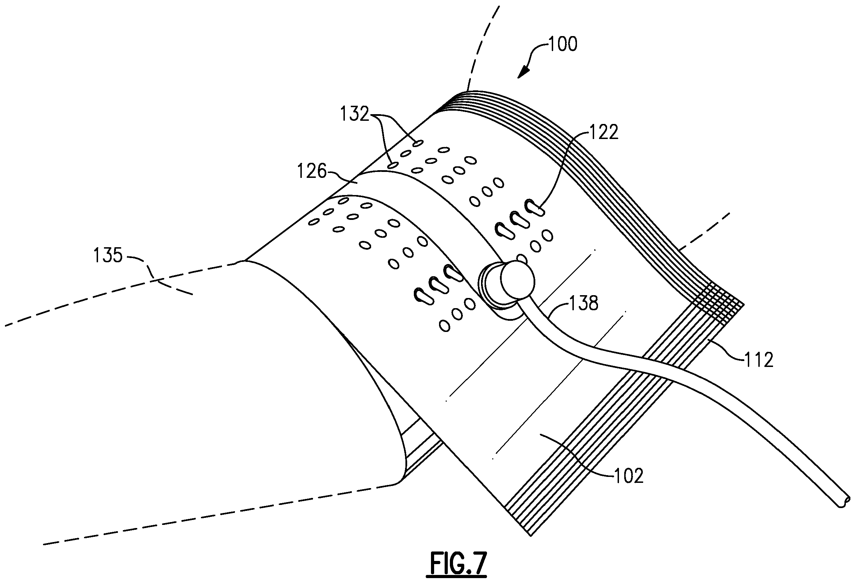

In use and referring to Fig. 7, the sleeve member 102 is wrapped about the limb (arm) 135 of a patient wherein the socket 110 extends through the slotted portion 126 and the angled raised protrusions 122 are aligned with and fitted to a row of the spaced openings 132. The slotted portion 126 is sized to only permit attachment to a range of patient limb sizes (circumferences). Pneumatic means 138 (shown partially) extending to a measuring apparatus (not shown) can be attached in releasable fashion to the socket 110 and the inflatable portion 114, Fig. 6, of the flexible sleeve member 102 can be inflated for measurement. Following use, the pneumatic means 138 are removed from the extending socket 110 and the protrusions 122 are removed from the openings 132. In one version, removal of the protrusions 122 from the openings 132 can cause the openings to tear and therefore disable re-use of the cuff.

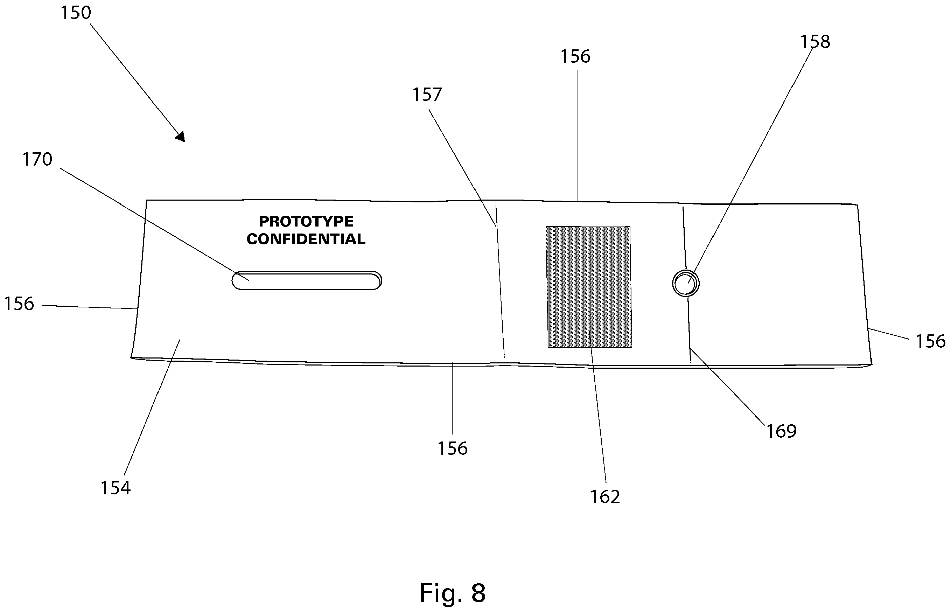

Referring to Figs. 8-11, there is disclosed an ecologically friendly blood pressure cuff 150 that is made in accordance with another exemplary embodiment. The cuff 150 according to this embodiment is made from at least one or a pair of planar sheets made from a biodegradable and highly flexible material, each sheet having a first side that is fluid impermeable and the remaining side being non-woven. According to the present embodiment, a pair of sheets 154 consisting of a fluid impermeable side made from polypropylene and a nonwoven side made from polyethylene that are sealed together to form a single sheet. One or two sheets 154 are used wherein the fluid impermeable sides (not shown) of each sheet are placed into adjacent relation to create a cuff interior and the nonwoven sides form an exterior in which all of the peripheral edges 156 are sealed by appropriate means, such as heat sealing, ultrasonic or RF welding. Alternatively, the entire cuff can be made from a single material, such as polypropylene, enabling recyclability. In this instance, an intermediate transverse seal 157 is also formed at the middle of the length dimension of the cuff 150, thereby dividing the cuff into two adjacent sections, one of which is capable of inflation as described herein.

An opening is formed in one of the planar sheets 154 in one of the sections wherein a port supported upon a smaller sheet section (not shown), preferably made from the same material as the sheets 154 is bonded to the interior of the sheet, and in which the port herein is defined by a socket 158 that extends through the opening wherein a fluid tight seal is created about the periphery of the socket within the opening. The socket 158 includes a relatively flexible circumferential or annular lip and is also preferably formed from the same material (i.e., polypropylene) as those constituting the sleeve sheets 154. A slotted portion 170 is formed in the opposite side of the cuff 150 wherein the major dimension of the slotted portion is aligned with the extending socket 158. According to this embodiment, the slotted portion 170 and opening/socket are provided at substantially the center of the width dimension of the cuff 150, as shown most clearly in Fig. 8.

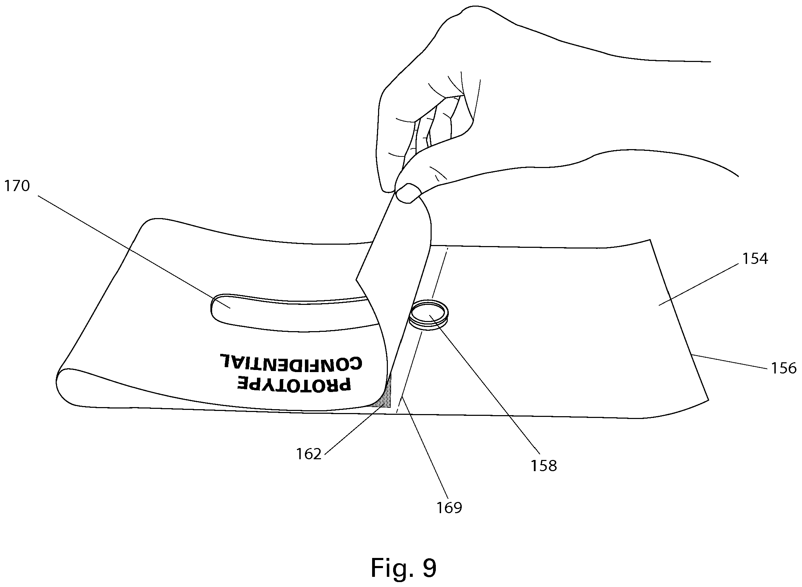

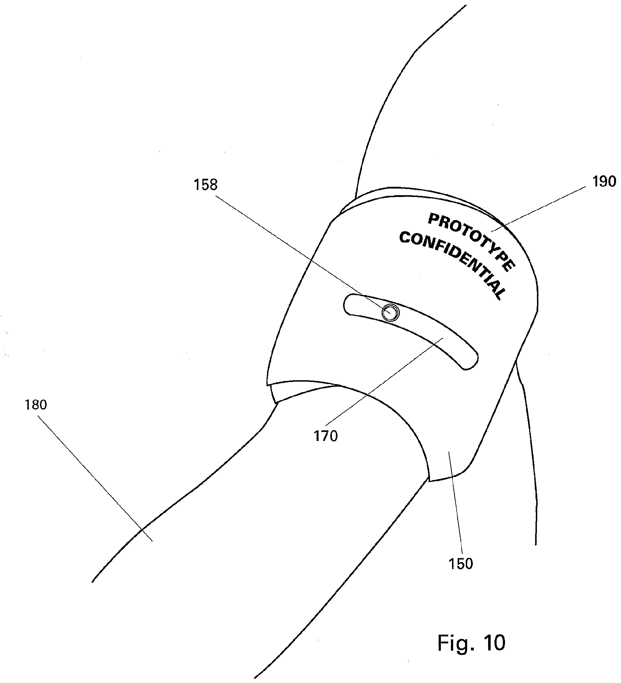

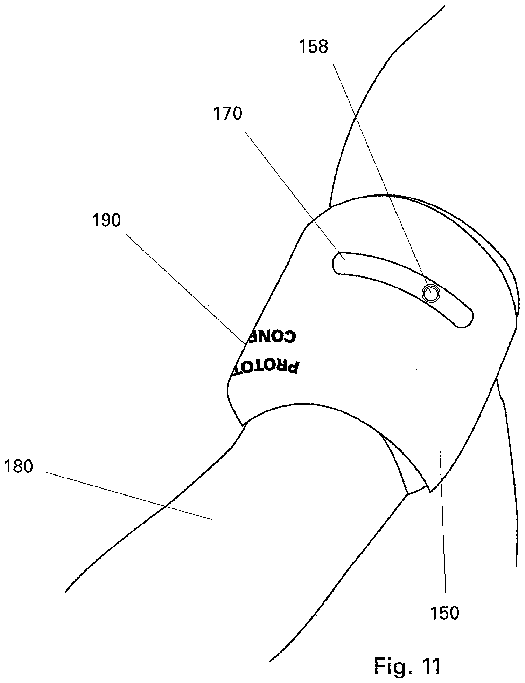

Adjacent the opening and extending socket 158, a hook fastener portion 162 is provided on the exterior of the cuff 150 on one side thereof. In this embodiment, the material of the cuff on the non-woven exterior of the sheets 154 is defined by a micro-structure that creates adhesion with the hook fastener portion 162 when the cuff 150 is wrapped, as shown, for example, according to Fig. 9. Due to the nature of this material, there is no need to provide a separate loop fastener portion to act as closure means for the cuff 150, when secured to the limb of a patient as shown, for example, in Figs. 10 and 11.

When wrapped, the slotted portion 170 is sized to accommodate the socket 158 and the slotted portion is further sized to be wrapped only within a predetermined range of limb (arm) circumferences. The slotted portion 170 in combination with the extending socket 158 serves numerous functions. First, and as noted the slotted portion 170 will only accommodate a predetermined range of arm circumferences, in which the slotted portion can be formed to accommodate a class of patient (e.g., a child, an adult, a large adult, etc). In addition, the slotted portion 170 serves as a guide to wrapping the cuff 150 about the limb 180, Fig. 10, given that the socket 158 must be fitted within the slotted portion when wrapped. Still further, the use of a slotted portion 170 and socket 158 permits the port to be flexibly located on the cuff 150 without interfering with the attachment, such as those involving hook and loop fasteners. In fact, the hook fastener portion 162 can also be positioned more conveniently along the exterior of the cuff 150 than in previously known cuffs. In this embodiment, the hook fastener portion 162 can be positioned more inboard (that is, inboard relative to the nearest lateral edge 156 of the cuff) such that attachment can occur within the overlapping portion of the cuff 150 that includes the slotted portion 170. By moving the attachment (fastener) portions more inboard, greater adhesion is achieved using less total surface area. Moreover and by selection of materials as in this embodiment, manufacture is simplified in that a separate loop fastener portion is not required given the inherent adhesive quality of the exterior sleeve material.

In this version, the durability of the material is affected with each attachment and subsequent removal of the cuff 150 from the hook fastener portion 162. This degradation of material influences the ability of the material to further adhere in those areas, thereby rendering the cuff 150 incapable of attachment after a finite number of uses. Depending on the material, this finite number of uses could be made to be one or several.

Referring to Figs. 10 and 11, the cuff 150 is shown in a wrapped condition about a limb 180 of a patient. For purposes of clarity, only the cuff 150 is shown and not the remainder of associated blood pressure apparatus. As seen from these views, the cuff can be wrapped in at least two separate wrapped orientations, see the relative position of a marker 190 denoted to indicate positional differences on the cuff 150 for clarity purposes. Either wrapped orientation is made possible due to the "on-center" positioning of the port/socket 158 and the slotted portion 170 since the port is positioned in the same location on the limb 180 of the patient in either instance. As such, neither orientation negatively affects the operation of the cuff 150 in obtaining a blood pressure measurement from the patient. In addition, the port itself due to the symmetrical positioning thereof on the sleeve can also be used as an arterial marker, eliminating the need to add a specific marker to the cuff 150.

By making the entire cuff as described herein from the same material, in this case polypropylene, the cuff can easily be recycled following use. It has been determined that the cuff is a #5 recyclable product when the entire cuff, including the port, are manufactured from the same material. In the case the cuff is made entirely from polyethylene it is a #2 or #4 recyclable product. Moreover and even in the instance in which the cuff is not made entirely from a homogenous material, the herein described sleeve when incinerated does not release toxic and ozone depleting gases as in the case of those sleeves containing polyvinylchloride (PVC). For those cuffs to which are treated with additives including those previously noted above, the cuffs are rendered biodegradable after a predetermined time period. In effect, the herein described cuffs are designed to be ecologically and environmentally friendly, whether through recallability or biodegradability.

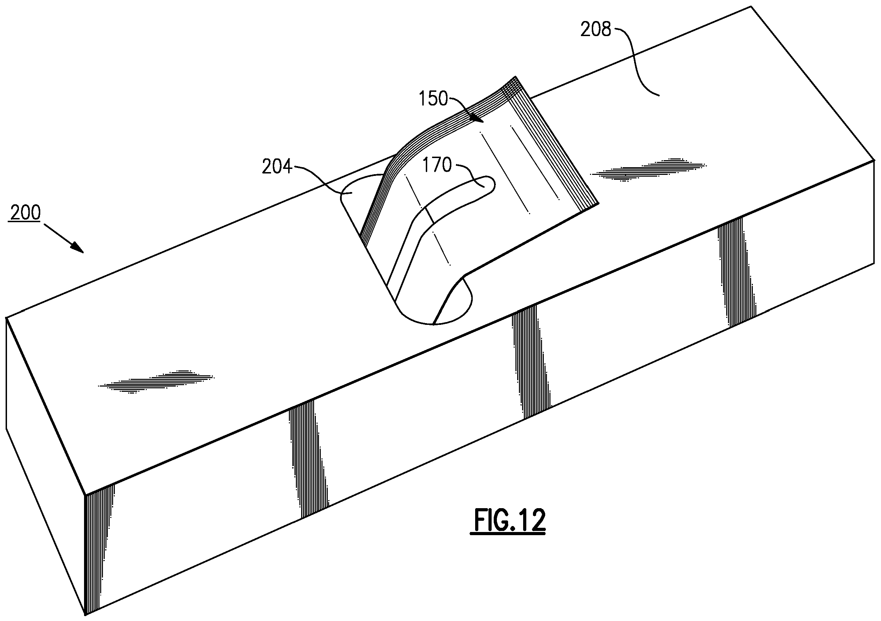

Referring to Fig. 12, the flexible nature of the herein described recyclable or biodegradable blood pressure cuffs enables additional versatility in the packaging and dispensing of same. By way of example, a cuff dispense container 200 can include a plurality of disposable, single patient or single patient-use cuffs, such as those made in accordance with Figs. 8 that are arranged in a stacked configuration. In one version, the cuffs 20 can be folded in half and nested one within the next succeeding cuff with the sockets facing the dispense slot of the container 200. It should be noted that other stacking configurations can easily be imagined, given the highly flexible nature of the herein described cuffs 150. The dispense container 200 according to this version is a cardboard or paper dispenser, which retains the contained stacked configuration of cuffs 150 (only one of which is shown), the container having a dispense slot 204 in a top or upper surface 208 of the container that is sized to dispense the cuffs individually (i.e. one at a time) to a user by pulling a portion of a cuff 150 extending therethrough in which the action of pulling a cuff for dispense pulls another succeeding cuff in the stacked configuration toward the slot 204. Though the dispenser 200 is shown herein for pulling cuffs 150 upwardly, it will be readily apparent that the dispenser can assume other attitudes and orientations (i.e., top slot facing downward, side or lateral dispense, etc.).

A plurality of cuffs, such as described above, can be otherwise stored for dispensing. For example, the herein described ecologically friendly cuffs can be disposed individually in a roll form such as described in U.S. Patent No. 5,819,739. Still other dispensing techniques can easily be imagined.

### PARTS LIST FOR FIGS. 1-12

- 20: cuff, recyclable or biodegradable
- 30: sleeve member, flexible
- 34: planar sheet
- 38: planar sheet
- 42: peripheral edges
- 45: seal, intermediate
- 46: interior
- 52: slotted region
- 60: bladder
- 64: socket
- 67: circumferential lip
- 68: opening
- 84: hose adapter
- 88: ports
- 100: cuff, recyclable
- 102: flexible sleeve member
- 104: planar sheet
- 108: planar sheet
- 110: socket
- 111: opening
- 112: peripheral edge seals
- 114: inflatable portion
- 115: intermediate seal
- 117: circumferential lip
- 118: structure, support
- 122: angled protrusions
- 126: slotted portion
- 132: openings, spaced
- 135: limb, patient
- 150: recyclable blood pressure cuff
- 154: sheets, planar
- 156: peripheral edge
- 157: transverse seal, edge
- 158: socket
- 162: hook fastener portion
- 169: artery marker
- 170: slotted portion
- 180: limb
- 190: marker, cuff
- 200: container, dispensing
- 204: slot, dispense
- 208: top surface

It will be readily apparent that other modifications and variations can be made in accordance with the aspects that are described herein and that the embodiments described are not intended to be exhaustive. For example, an ecologically friendly cuff can also be made using a combination of polyethylene and polypropylene including a polyethylene interior and the non- woven polypropylene exterior previously described with regard to cuff 150. Other suitable versions within the teachings provided herein should easily be contemplated.

## Claims

1. A blood pressure cuff (20; 100; 150) comprising:
a flexible sleeve (30; 102) having an envelope-like structure made up of a stacked pair of planar sheets (34, 38; 104, 108; 154) sealed along all peripheral edges (42; 112; 156), the planar sheets having a length dimension that is significantly larger than a corresponding width dimension,
wherein the flexible sleeve (30; 102) includes an inflatable portion (60, 114) within one side of the sleeve (30; 102) with respect to the length dimension;
an exterior port in the form of a socket (64; 110; 158) extending from an exterior surface of said inflatable portion (60; 114) and communicating fluidly with the interior of said inflatable portion; and
a slotted portion (52; 126; 170) formed through each of the planar sheets on the other side of the sleeve (30; 102) opposite said one side with respect to the length dimension, the slotted portion (52; 126; 170) having a major dimension aligned with the length dimension, wherein the slotted portion (52; 126; 170) is sized for receiving said port when said sleeve (30; 102) is circumferentially wrapped about a limb (135; 180) of a patient, said slotted portion (52; 126; 170) being sized to limit the range of limb sizes to which the cuff (20; 100; 150) can be attached with the port extending through the slotted portion (52; 126; 170).

2. A cuff (20; 100; 150) as recited in Claim 1, wherein said slotted portion (52; 126; 170) and said port are aligned with one another, each being disposed at substantially the center of the width dimension of said sleeve (30; 102).

3. A cuff (20; 100; 150) as recited in Claim 2, wherein said cuff (20; 100; 150) can be wrapped in at least two opposite orientations about the limb of a patient and in which said port is in the same location relative to said patient in each orientation.

4. A cuff (20; 100; 150) as recited in Claim 1, including closure means for releasably securing said cuff (20; 100; 150) in said circumferentially wrapped configuration.

5. A cuff (20; 100; 150) as recited in Claim 4, wherein said closure means includes hook and loop fasteners.

6. A cuff (20; 100; 150) as recited in Claim 5, wherein the exterior of said sleeve (30; 102) is made from a material that enables adhesion relative to a hook fastener portion provided on the exterior of one of said planar sheets of said sleeve (30; 102)

7. A cuff (20; 100; 150) as recited in Claim 1, wherein the entire cuff (20; 100; 150) is made from a single material, enabling said cuff (20; 100; 150) to be recycled.

8. A method for manufacturing a blood pressure cuff (20; 100; 150), said method comprising the steps of:
providing a flexible sleeve (30; 102) having an envelope-like structure made up of a stacked pair of planar sheets (34, 38; 104, 108; 154) sealed along all peripheral edges (42; 112; 156), the planar sheets having a length dimension that is significantly larger than a corresponding width dimension, the flexible sleeve (30; 102) including an inflatable portion (60, 114) within one side of the sleeve (30; 102) with respect to the length dimension;
adding an external port in the form of a socket (64; 110; 158) to said sleeve (30; 102), said external port extending from an exterior surface of the sleeve and including an opening (68; 111) in fluid communication with the inflatable portion (60; 114) of said sleeve (30; 102); and
creating a slotted portion (52; 126; 170) in the other side of said sleeve (30; 102) opposite from said one side with respect to the length dimension and formed through each of the planar sheets, wherein said slotted portion (52; 126; 170) has a major dimension aligned with the length dimension, wherein the slotted portion (52; 126; 170) is sized to receive the external port when the cuff (20; 100; 150) is wrapped about the limb (135; 180) of a patient, said slotted portion (52; 126; 170) being sized in order to limit the range of limb sizes to which said cuff (20; 100; 150) can be attached with the external port extending through the slotted portion (52; 126; 170).

9. A method as recited in Claim 8, wherein said sleeve (30; 102) is entirely made from a recyclable material.

10. A method as recited in Claim 8, wherein said slotted portion (52; 126; 170) is sized to permit the attachment of pneumatic means to said port while extending through the slotted portion (52; 126; 170) when the cuff is wrapped about the limb (135; 180) of a patient to permit inflation and deflation of said sleeve (30; 102).

11. A method as recited in Claim 8, including the step of positioning said port and said slotted portion (52; 126; 170) at substantially the center of the width dimension of said sleeve (30; 102).

12. A method as recited in Claim 11, wherein said cuff (20; 100; 150) is capable of being wrapped in at least two opposite orientations about the limb (135; 180) of a patient in which the port is in the same location relative to the patient in each orientation.

13. A method as recited in Claim 8, including the step of providing closure means for releasably securing the sleeve (30; 102) in a wrapped configuration.

14. A method as recited in claim 13 , wherein said closure means includes hook and loop fasteners wherein the exterior surface of the sleeve (30; 102) is made from a material that provides adhesion to a hook fastener portion of the sleeve (30; 102), thereby serving as loop fasteners.

## Patentansprüche

1. Eine Blutdruckmanschette (20; 100; 150), umfassend:
einen flexiblen Schlauch (30; 102) mit einer umschlagähnlichen Struktur, der aus einem übereinandergelegten Paar von flachen Lagen (34, 38; 104, 108; 154) besteht, die entlang aller randständigen Kanten (42; 112; 156) versiegelt sind, wobei die flachen Lagen ein Längenmaß aufweisen, das erheblich größer als ein entsprechendes Breitenmaß ist, wobei der flexible Schlauch (30; 102) einen aufblasbaren Teil (60, 114) innerhalb einer Seite des Schlauchs (30; 102) in Bezug auf das Längenmaß einschließt;
einen Außenanschluss in Form einer Buchse (64; 110; 158), der sich von einer Außenfläche des genannten aufblasbaren Teils (60; 114) erstreckt und fließend mit dem Inneren des genannten aufblasbaren Teils kommuniziert; und
einen Schlitzteil (52; 126; 170), der durch jede der flachen Lagen auf der anderen Seite des Schlauchs (30; 102) gegenüber der genannten einen Seite in Bezug auf das Längenmaß gebildet wird, wobei der Schlitzteil (52; 126; 170) ein größeres Maß aufweist, das am Längenmaß ausgerichtet ist, wobei der Schlitzteil (52; 126; 170) für die Aufnahme des genannten Anschlusses dimensioniert ist, wenn der genannte Schlauch (30;102) um eine Extremität (135; 180) eines Patienten herum gewickelt wird, wobei der genannte Schlitzteil (52; 126; 170) so dimensioniert ist, dass der Bereich der Extremitätengrößen begrenzt ist, in dem die Manschette (20; 100; 150) mit dem durch den Schlitzteil (52; 126; 170) reichenden Anschluss angebracht werden kann.

2. Eine Manschette (20; 100; 150) nach Anspruch 1, wobei der genannte Schlitzteil (52; 126; 170) und der genannte Anschluss aufeinander ausgerichtet sind und sich dabei jeweils überwiegend in der Mitte des Breitenmaßes der genannten Schlauchs (30; 102) befinden.

3. Eine Manschette (20; 100; 150) nach Anspruch 2, wobei die genannte Manschette (20; 100; 150) in mindestens zwei entgegengesetzten Richtungen um die Extremität eines Patienten gewickelt werden kann und bei der sich der genannte Anschluss in jeder Richtung an derselben Stelle bezüglich des genannten Patienten befindet.

4. Eine Manschette (20; 100; 150) nach Anspruch 1, einschließlich einer Verschlussmöglichkeit für die lösbare Befestigung der genannten Manschette (20; 100; 150) in der genannten herumgewickelten Konfiguration.

5. Eine Manschette (20; 100; 150) nach Anspruch 4, wobei die genannte Verschlussmöglichkeit Klettverschlüsse einschließt.

6. Eine Manschette (20; 100; 150) nach Anspruch 5, wobei die Außenseite des genannten Schlauchs (30; 102) aus einem Material besteht, das eine Haftung an einem Hakenbefestigungsteil ermöglicht, das an der Außenseite von einer der genannten flachen Lagen des genannten Schlauchs (30; 102) bereitgestellt wird.

7. Eine Manschette (20; 100; 150) nach Anspruch 1, wobei die gesamte Manschette (20; 100; 150) aus einem einzigen Material besteht, so dass die genannte Manschette (20; 100; 150) recycelt werden kann.

8. Ein Verfahren zur Herstellung einer Blutdruckmanschette (20; 100; 150), wobei das Verfahren die Schritte umfasst:
Bereitstellen eines flexiblen Schlauchs (30; 102) mit einer umschlagähnlichen Struktur, die aus einem übereinandergelegten Paar von flachen Lagen (34, 38; 104, 108; 154) besteht, die entlang aller randständigen Kanten (42; 112; 156) versiegelt sind, wobei die flachen Lagen ein Längenmaß aufweisen, das erheblich größer als ein entsprechendes Breitenmaß ist, wobei der flexible Schlauch (30; 102) einen aufblasbaren Teil (60, 114) innerhalb einer Seite des Schlauchs (30; 102) in Bezug auf das Längenmaß einschließt;
Hinzufügen eines Außenanschlusses in Form einer Buchse (64; 110; 158) zu dem genannten Schlauch (30; 102), wobei sich der Außenanschluss von einer Außenfläche des Schlauchs erstreckt und eine Öffnung (68; 111) in fließender Kommunikation mit dem aufblasbaren Teil (60; 114) des genannten Schlauchs (30; 102) einschließt; und
Schaffen eines Schlitzteils (52; 126; 170) auf der anderen Seite des genannten Schlauchs (30; 102) gegenüber von der genannten einen Seite in Bezug auf das Längenmaß und durch jede der flachen Lagen gebildet, wobei der genannte Schlitzteil (52; 126; 170) ein größeres Maß aufweist, das am Längenmaß ausgerichtet ist, wobei der Schlitzteil (52; 126; 170) für die Aufnahme des Außenanschlusses dimensioniert ist, wenn die Manschette (20; 100; 150) um die Extremität (135; 180) eines Patienten gewickelt wird,
wobei der genannte Schlitzteil (52; 126; 170) so dimensioniert ist, dass der Bereich der Extremitätengrößen begrenzt ist, in dem die genannte Manschette (20; 100; 150) mit dem durch den Schlitzteil (52; 126; 170) reichenden Außenanschluss angebracht werden kann.

9. Ein Verfahren nach Anspruch 8, wobei der genannte Schlauch (30; 102) vollständig aus recycelbarem Material besteht.

10. Ein Verfahren nach Anspruch 8, wobei der genannte Schlitzteil (52; 126; 170) so dimensioniert ist, dass pneumatische Mittel an dem genannten Anschluss befestigt werden können, während er durch den Schlitzteil (52; 126; 170) reicht, wenn die Manschette um die Extremität (135; 180) eines Patienten gewickelt ist, um ein Aufpumpen und Entleeren des genannten Schlauchs (30; 102) zuzulassen.

11. Ein Verfahren nach Anspruch 8, einschließlich des Schritts der Positionierung des genannten Anschlusses und des genannten Schlitzteils (52; 126; 170) überwiegend in der Mitte des Breitenmaßes der genannten Schlauchs (30; 102).

12. Ein Verfahren nach Anspruch 11, wobei die genannte Manschette (20; 100; 150) in mindestens zwei entgegengesetzten Richtungen um die Extremität (135; 180) eines Patienten gewickelt werden kann, bei der sich der Anschluss in jeder Richtung an derselben Stelle bezüglich des genannten Patienten befindet.

13. Ein Verfahren nach Anspruch 8, einschließlich des Schritts der Bereitstellung einer Verschlussmöglichkeit für die lösbare Befestigung des Schlauchs (30; 102) in einer herumgewickelten Konfiguration.

14. Ein Verfahren nach Anspruch 13, wobei die genannte Verschlussmöglichkeit Klettverschlüsse einschließt, wobei die Außenfläche des Schlauchs (30; 102) aus einem Material besteht, das eine Haftung an einem Hakenbefestigungsteil des Schlauchs (30; 102) bereitstellt und dadurch als Schlaufenbefestigungen dient.

## Revendications

1. Brassard de tensiomètre (20 ; 100 ; 150) comprenant :
un manchon souple (30 ; 102) ayant une structure en forme d'enveloppe constituée d'une paire de feuilles plates empilées (34, 38 ; 104, 108 ; 154) scellées sur tous les bords périphériques (42 ; 112 ; 156), les feuilles plates ayant une dimension en longueur qui est significativement plus grande qu'une dimension en largeur correspondante, dans lequel le manchon souple (30 ; 102) inclut une partie gonflable (60 ; 114) à l'intérieur d'un côté du manchon (30 ; 102) par rapport à la dimension en longueur ;
un orifice extérieur en forme de douille (64 ; 110 ; 158) s'étendant d'une surface extérieure de ladite partie gonflable (60 ; 114), et communiquant de manière fluidique avec l'intérieur de ladite partie gonflable ; et
une partie fendue (52 ; 126 ; 170) formée au travers de chacune des feuilles plates sur l'autre côté du manchon (30 ; 102) en face dudit un côté par rapport à la dimension en longueur, la partie fendue (52 ; 126 ; 170) ayant une dimension importante alignée avec la dimension en longueur, dans lequel la partie fendue (52 ; 126 ; 170) est dimensionnée pour recevoir ledit orifice lorsque ledit manchon (30 ; 102) est enroulé sur sa circonférence autour d'un membre (135 ; 180) d'un patient, ladite partie fendue (52 ; 126 ; 170) étant dimensionnée pour limiter la plage de tailles de membre auquel le brassard (20 ; 100 ; 150) peut être attaché avec l'orifice s'étendant dans la partie fendue (52 ; 126 ; 170).

2. Brassard (20 ; 100 ; 150) selon la revendication 1, dans lequel ladite partie fendue (52 ; 126 ; 170) et ledit orifice sont alignés l'un avec l'autre, chacun étant disposé essentiellement au centre de la dimension en largeur dudit manchon (30, 102).

3. Brassard (20 ; 100 ; 150) selon la revendication 2, dans lequel ledit brassard (20 ; 100 ; 150) peut être enroulé dans au moins deux sens opposés autour du membre d'un patient et dans lequel ledit orifice est dans la même position par rapport audit patient dans chaque sens.

4. Brassard (20 ; 100 ; 150) selon la revendication 1, incluant des moyens de fermeture destinés à fixer de manière détachable ledit brassard (20 ; 100 ; 150) dans ladite configuration enroulée sur sa circonférence.

5. Brassard (20 ; 100 ; 150) selon la revendication 4, dans lequel les moyens de fermeture incluent des fixations auto-agrippantes.

6. Brassard (20 ; 100 ; 150) selon la revendication 5, dans lequel l'extérieur dudit manchon (30, 102) est constitué d'un matériau qui permet l'adhérence à une partie de fixation à crochet pourvue sur l'extérieur de l'une desdites feuilles plates dudit manchon (30 ; 102).

7. Brassard (20 ; 100 ; 150) selon la revendication 1, dans lequel l'ensemble du brassard (20 ; 100 ; 150) est fait à partir d'un seul matériau, ce qui permet audit brassard (20 ; 100 ; 150) d'être recyclé.

8. Procédé de fabrication d'un brassard de tensiomètre (20 ; 100 ; 150), ledit procédé comprenant les étapes suivantes :
fournir un manchon souple (30 ; 102) ayant une structure en forme d'enveloppe constituée d'une paire de feuilles plates empilées (34, 38 ; 104, 108 ; 154) scellées sur tous les bords périphériques (42 ; 112 ; 156), les feuilles plates ayant une dimension en longueur qui est significativement plus grande qu'une dimension en largeur correspondante, le manchon souple (30 ; 102) incluant une partie gonflable (60 ; 114) à l'intérieur d'un côté du manchon (30 ; 102) par rapport à la dimension en longueur ;
ajouter un orifice externe en forme de douille (64 ; 110 ; 158) audit manchon (30 ; 102), ledit orifice externe s'étendant d'une surface extérieure du manchon et incluant une ouverture (68 ; 111) en communication fluidique avec la partie gonflable (60 ; 114) dudit manchon (30 ; 102) ; et
créer une partie fendue (52 ; 126 ; 170) dans l'autre côté dudit manchon (30 ; 102) en face dudit un côté par rapport à la dimension en longueur et formée au travers de chacune des feuilles plates, dans lequel ladite partie fendue (52 ; 126 ; 170) a une dimension importante alignée avec la dimension en longueur, dans lequel la partie fendue (52 ; 126 ; 170) est dimensionnée pour recevoir l'orifice externe lorsque le brassard (20 ; 100 ; 150) est enroulé autour du membre (135 ; 180) d'un patient, ladite partie fendue (52 ; 126 ; 170) étant dimensionnée afin de limiter la plage des tailles de membre auquel ledit brassard (20 ; 100 ; 150) peut être fixé avec l'orifice externe s'étendant dans la partie fendue (52 ; 126 ; 170).

9. Procédé selon la revendication 8, dans lequel ledit manchon (30 ; 102) est entièrement fait à partir d'un matériau recyclable.

10. Procédé selon la revendication 8, dans lequel ladite partie fendue (52 ; 126 ; 170) est dimensionnée pour permettre la fixation des moyens pneumatiques audit orifice tout en s'étendant à travers la partie fendue (52 ; 126 ; 170) lorsque le brassard est enroulé autour du membre (135 ; 180) d'un patient pour permettre le gonflage et le dégonflage dudit manchon (30 ; 102).

11. Procédé selon la revendication 8, incluant l'étape consistant à positionner ledit orifice et ladite partie fendue (52 ; 126 ; 170) essentiellement au centre de la dimension en largeur dudit manchon (30 ; 102).

12. Procédé selon la revendication 11, dans lequel le brassard (20 ; 100 ; 150) peut être enroulé dans au moins deux sens opposés autour du membre (135 ; 180) d'un patient dans lesquels l'orifice est dans la même position par rapport au patient dans chaque sens.

13. Procédé selon la revendication 8, incluant l'étape consistant à fournir des moyens de fermeture pour la fixation de manière détachable du manchon (30 ; 102) dans une configuration enroulée.

14. Procédé selon la revendication 13, dans lequel les moyens de fermeture incluent des fixations auto-agrippantes dans lesquelles la surface extérieure du manchon (30 ; 102) est constituée d'un matériau qui permet l'adhérence à une partie de fixation à crochet du manchon (30 ; 102), servant ainsi de fixations à boucle.
